(19) Europäisches Patentamt
European Patent Office
Office européen des brevets

(11) **EP 4 782 419 A1**

(12) **EUROPEAN PATENT APPLICATION**
published in accordance with Art. 153(4) EPC

(43) Date of publication:
**29.07.2026 Bulletin 2026/31**

(21) Application number: **24868383.1**

(22) Date of filing: **20.09.2024**

(51) International Patent Classification (IPC):
*C04B 35/486* (2006.01)  *A61C 13/083* (2006.01)
*A61K 6/818* (2020.01)  *A61K 6/824* (2020.01)
*C01G 25/02* (2006.01)

(52) Cooperative Patent Classification (CPC):
A61C 13/083; A61K 6/818; A61K 6/824;
C01G 25/02; C04B 35/486

(86) International application number:
**PCT/JP2024/033776**

(87) International publication number:
**WO 2025/063307 (27.03.2025 Gazette 2025/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.09.2023 JP 2023159085**

(71) Applicant: Kuraray Noritake Dental Inc.
**Kurashiki-shi
Okayama 710-0801 (JP)**

(72) Inventors:
• **KAWAI, Makito**
  **Miyoshi-shi, Aichi 4700293 (JP)**
• **NISHIMURA, Takumi**
  **Miyoshi-shi, Aichi 4700293 (JP)**
• **NAKANO, Kirihiro**
  **Miyoshi-shi, Aichi 4700293 (JP)**
• **SAKAMOTO, Hiroyuki**
  **Miyoshi-shi, Aichi 4700293 (JP)**
• **YAMAMOTO, Takahisa**
  **Nagoya-shi, Aichi 4648601 (JP)**

(74) Representative: **D Young & Co LLP
3 Noble Street
London EC2V 7BQ (GB)**

(54) **ZIRCONIA COMPOSITION AND METHOD FOR PRODUCING SAME**

(57) The present invention provides a zirconia composition that can yield a zirconia sintered body with excellent mechanical strength while allowing its opalescence to be controlled within an appropriate range, and a method for producing such a zirconia composition. The present invention relates to a zirconia composition comprising: zirconia particles with a zirconia concentration of 80 mass% or more; and small particles with a stabilizer concentration of 10 to 50 mass%, wherein the small particles have a primary particle diameter smaller than the average primary particle diameter of the zirconia particles.

**Description**

TECHNICAL FIELD

The present invention relates to a zirconia composition and a method for producing same. More specifically, the present invention relates to a zirconia composition that can yield a zirconia sintered body with excellent mechanical strength while allowing its opalescence to be controlled within an appropriate range, and to a method for producing such a zirconia composition.

BACKGROUND ART

Zirconia sintered bodies find widespread applications in industry. In particular, zirconia sintered bodies have recently been used in dental material applications such as dental prostheses. In many cases, such dental prostheses are produced from a zirconia molded body of a desired shape, such as a disc or a prism, prepared by, for example, press molding zirconia particles or molding a composition containing zirconia particles. The zirconia molded body is then pre-sintered to prepare a pre-sintered body (mill blank), which is subsequently fired after being cut (milled) into a shape of the desired dental prosthesis.

Zirconia is a compound that undergoes a phase transformation between crystal systems. In this connection, partially-stabilized zirconia (PSZ) and fully-stabilized zirconia (FSZ), which exist as solid solutions of zirconia with stabilizers such as yttria (yttrium oxide; $Y_2O_3$) to prevent such phase transformations, are used in a wide variety of fields.

In the field of dentistry, zirconia material has been used as a material for frameworks because zirconia material, while possessing high strength, exhibits low translucency. In recent years, however, improvements in the translucency of zirconia material have led to increased production of zirconia-only dental prostheses.

An example of a dental material using such a zirconia material can be found in, for example, Patent Literature 1.

CITATION LIST

Patent Literature

Patent Literature 1: WO2022/138881

SUMMARY OF INVENTION

Technical Problem

However, a zirconia sintered body obtained by using the zirconia pre-sintered body according to Patent Literature 1 poses an issue when the crystalline structure is simply held small. Specifically, due to the uniform structure, it selectively reflects or transmits certain wavelengths, resulting in the manifestation of color, commonly referred to as structural color (hereinafter, this characteristic will be referred to as "opalescence"). In this respect, there remain areas for improvement in terms of achieving an appearance more closely matching the color tone of natural teeth.

It is an object of the present invention to provide a zirconia composition that can yield a zirconia sintered body with excellent mechanical strength while allowing its opalescence to be controlled within an appropriate range, and a method for producing such a zirconia composition.

Solution to Problem

The present inventors conducted extensive research to find a solution to the foregoing issue, and found that a zirconia sintered body having excellent mechanical strength and with its opalescence controlled within an appropriate range can be obtained from a zirconia composition that comprises zirconia particles with a zirconia concentration of 80 mass% or more, and small particles with a concentration of 10 to 50 mass% of a stabilizer capable of preventing a phase transformation of zirconia, and in which the small particles have a primary particle diameter smaller than the average primary particle diameter of the zirconia particles. Building upon this finding, the present inventors conducted additional examinations, ultimately resulting in the completion of the present invention.

The present invention includes the following.

[1] A zirconia composition comprising:

zirconia particles with a zirconia concentration of 80 mass% or more; and

small particles with a concentration of 10 to 50 mass% of a stabilizer capable of preventing a phase transformation of zirconia,
wherein the small particles have a primary particle diameter smaller than the average primary particle diameter of the zirconia particles.

[2] The zirconia composition according to [1], wherein the small particles with a stabilizer concentration of 10 to 50 mass% represent six or more of ten small particles arbitrarily selected under observation by TEM.

[3] The zirconia composition according to [1] or [2], wherein the average primary particle diameter of the zirconia particles is 0.06 to 0.17 $\mu$m.

[4] The zirconia composition according to any one of [1] to [3], wherein the small particles have an average primary particle diameter of less than 0.06 $\mu$m.

[5] The zirconia composition according to any one of [1] to [4], wherein the stabilizer capable of preventing a phase transformation of zirconia is yttria.

[6] The zirconia composition according to any one of [1] to [5], wherein the area ratio of the zirconia particles and the small particles is 55/45 to 99/1 under observation by TEM.

[7] A method for producing a zirconia composition of any one of [1] to [6], comprising the steps of:

producing a raw material composition comprising zirconia particles, and particles of a stabilizer capable of preventing a phase transformation of zirconia; and
pulverizing the zirconia composition,
wherein the pulverization step employs a pulverization medium of less than 1 mm in diameter.

[8] The method for producing a zirconia composition according to [7], wherein the pulverization step involves a processing time of 20 minutes to 2 hours.

[9] The method for producing a zirconia composition according to [7] or [8], wherein the average primary particle diameter of the zirconia particles is 0.06 to 0.17 $\mu$m.

[10] The method for producing a zirconia composition according to any one of [7] to [9], wherein the small particles have an average primary particle diameter of less than 0.06 $\mu$m.

[11] The method for producing a zirconia composition according to any one of [7] to [10], wherein the stabilizer capable of preventing a phase transformation of zirconia is yttria.

[12] The method for producing a zirconia composition according to any one of [7] to [11], which further comprises the step of producing a slurry comprising the raw material composition and granulating the slurry by spray drying.

Advantageous Effects of Invention

According to the present invention, a zirconia composition can be provided that can yield a zirconia sintered body with excellent mechanical strength while allowing its opalescence to be controlled within an appropriate range, as well as a method for producing such a zirconia composition.

By using a zirconia pre-sintered body of the present invention, a zirconia sintered body can be provided that exhibits excellent mechanical strength and translucency, even with a highest sintering temperature (for example, 1,450°C or less) lower than that conventionally employed.

Furthermore, by using a zirconia pre-sintered body of the present invention, a zirconia sintered body that exhibits excellent mechanical strength and translucency can be provided without employing a HIP apparatus.

DESCRIPTION OF EMBODIMENTS

[Zirconia Composition]

A zirconia composition of the present invention comprises:

zirconia particles with a zirconia concentration of 80 mass% or more; and
small particles with a concentration of 10 to 50 mass% of a stabilizer capable of preventing a phase transformation of zirconia (hereinafter, also referred to simply as "small particles"),
wherein the small particles have a primary particle diameter smaller than the average primary particle diameter of the zirconia particles.

As used herein, a "zirconia composition" refers to a composition comprising a zirconia powder and a stabilizer powder.

As used herein, a "molded body" refers to an object that has not reached a semi-sintered state (pre-sintered state) or a sintered state. That is, "molded body" distinguishes itself from pre-sintered body and sintered body in that it remains unfired after being molded.

As used herein, a "zirconia pre-sintered body" refers to an object in a semi-sintered state where zirconia particles have formed necks between particles but have not fully sintered.

As used herein, a "zirconia sintered body" refers to an object in a sintered state where zirconia particles have fully sintered. In a zirconia sintered body, sintering has caused zirconia particles to consolidate, increasing the relative density and promoting densification, resulting in a fully sintered state with a relative density of 95% or more.

As used herein, "zirconia" refers to zirconium(IV) oxide ($ZrO_2$), with $ZrO_2$ particles containing a trace amount (0.5 mass% or more and 3 mass% or less) of $HfO_2$ relative to the amount of $ZrO_2$. Because $HfO_2$ is difficult to separate, terms such as "zirconia", "zirconia particles", and "zirconia powder" are intended to include both $ZrO_2$ and $HfO_2$. Likewise, particles or powders with the stabilizer present in zirconia as a solid solution are also encompassed by "zirconia particles" or "zirconia powders."

As used herein, the term "ordinary pressure" means standard atmospheric pressure (1 atm).

In this specification, the upper limits and lower limits of numeric ranges (for example, such as temperature ranges, the content of each component, the abundance of crystal systems, values calculated from components, and various physical properties) can be appropriately combined.

The exact reason remains unclear as to why a zirconia composition of the present invention can yield a zirconia sintered body with excellent mechanical strength while allowing its opalescence to be controlled within an appropriate range by the presence of 10 to 50 mass% of a stabilizer capable of preventing a phase transformation of zirconia in the small particles contained with the zirconia particles. However, the following speculation has been made.

By incorporating small particles with a concentration of 10 to 50 mass% of a stabilizer capable of preventing a phase transformation of zirconia in a zirconia composition containing zirconia particles, it was possible to improve the evenness of the stabilizer distribution in the zirconia composition-a feature not achievable with conventional zirconia not forming a solid solution (zirconia not in solid solution with a stabilizer). This is believed to have led to a relatively uniform sintered structure in terms of crystal grain size and composition in the resulting sintered body, thereby achieving excellent mechanical strength in the sintered body with its opalescence controlled within an appropriate range.

[Production Method of Zirconia Composition]

A zirconia composition of the present invention can be produced through a pulverization process performed under predetermined conditions by providing energy to a raw material zirconia composition (or simply "raw material composition" or "unpulverized zirconia composition") comprising zirconia particles and particles of a stabilizer capable of preventing a phase transformation of zirconia (hereinafter, also referred to simply as "stabilizer"), in excess of the energy conventionally employed.

Examples of the stabilizer capable of preventing a phase transformation of zirconia (hereinafter, also referred to simply as "stabilizer") include oxides such as calcium oxide (CaO), magnesium oxide (MgO), yttrium oxide ($Y_2O_3$), cerium oxide ($CeO_2$), scandium oxide ($Sc_2O_3$), niobium oxide ($Nb_2O_5$), lanthanum oxide ($La_2O_3$), erbium oxide ($Er_2O_3$), praseodymium oxide ($Pr_2O_3$, $Pr_6O_{11}$), samarium oxide ($Sm_2O_3$), europium oxide ($Eu_2O_3$), thulium oxide ($Tm_2O_3$), gallium oxide ($Ga_2O_3$), indium oxide ($In_2O_3$), and ytterbium oxide ($Yb_2O_3$). Preferred is yttria ($Y_2O_3$). The stabilizer may be used alone, or two or more thereof may be used in combination.

In a zirconia composition of the present invention, the stabilizer may be solely yttria ($Y_2O_3$). Alternatively, a zirconia composition of the present invention may comprise another stabilizer, other than yttria, capable of preventing a phase transformation of zirconia, in addition to yttria.

The stabilizer content in the zirconia composition is preferably 2.5 mol% or more, more preferably 3.0 mol% or more, even more preferably 3.5 mol% or more, particularly preferably 4.0 mol% or more relative to the total moles of zirconia and the stabilizer. A stabilizer content of 2.5 mol% or more is preferred in terms of increasing the cubic zirconia within the crystal systems of the sintered body and improving translucency.

The stabilizer content is preferably 10 mol% or less, more preferably 9.0 mol% or less, even more preferably 8.5 mol% or less, particularly preferably 8 mol% or less. A stabilizer content of 10 mol% or less is preferred in terms of preventing a decrease in strength.

The following describes a method for producing the zirconia composition through the case where the stabilizer is yttria.

The unpulverized zirconia composition (raw material composition) can be produced by mixing the raw material zirconia powder and yttria powder. The mixing method is not particularly limited, and any known method and apparatus may be used.

The zirconia and yttria particles constituting their respective powders may be prepared using a method, for example, such as the breakdown process, in which coarse particles are pulverized or crushed into a fine powder, or the building-up process, which synthesizes particles through nucleation and growth from atoms or ions. However, in view of ease of production, the preferred method is the breakdown process from consideration that the zirconia composition produced via the building-up process still requires a predetermined pulverization process to adjust the stabilizer concentration in the small particles within the desired range.

The following describes a method of production of the zirconia composition, taking the breakdown process as an example.

Preferably, the zirconia constituting the zirconia powder comprises monoclinic zirconia.

In a certain embodiment, the zirconia constituting the zirconia powder may comprise tetragonal zirconia and/or cubic zirconia, in addition to monoclinic zirconia.

In another embodiment, the zirconia constituting the zirconia powder may comprise tetragonal zirconia and/or cubic zirconia, without monoclinic zirconia.

In the zirconia powder, these crystal systems may be present alone, or two or more thereof may be present in combination.

The raw material zirconia powder may employ commercially available zirconia particles, or a commercially available powder may be used after pulverization with a known pulverizing mixer (such as a ball mill).

The raw material yttria powder may employ commercially available yttria particles, or a commercially available powder may be used after pulverization with a known pulverizing mixer (such as a ball mill).

Examples of commercially available zirconia powders include zirconia powder products such as those manufactured by Tosoh Corporation under various trade names, including Zpex® ($Y_2O_3$ content: 3 mol%), Zpex® 4 ($Y_2O_3$ content: 4 mol%), Zpex® Smile® ($Y_2O_3$ content: 5.5 mol%), TZ-3Y ($Y_2O_3$ content: 3 mol%), TZ-3YS ($Y_2O_3$ content: 3 mol%), TZ-4YS ($Y_2O_3$ content: 4 mol%), TZ-6Y ($Y_2O_3$ content: 6 mol%), TZ-6YS ($Y_2O_3$ content: 6 mol%), TZ-8YS ($Y_2O_3$ content: 8 mol%), TZ-10YS ($Y_2O_3$ content: 10 mol%), TZ-3Y-E ($Y_2O_3$ content: 3 mol%), TZ-3YS-E ($Y_2O_3$ content: 3 mol%), TZ-3YB-E ($Y_2O_3$ content: 3 mol%), TZ-3YSB-E ($Y_2O_3$ content: 3 mol%), TZ-3YB ($Y_2O_3$ content: 3 mol%), TZ-3YSB ($Y_2O_3$ content: 3 mol%), TZ-3Y20AB ($Y_2O_3$ content: 3 mol%), TZ-8YSB ($Y_2O_3$ content: 8 mol%), and TZ-0 ($Y_2O_3$ content: 0 mol%; monoclinic zirconia).

Subsequently, the zirconia composition comprising zirconia particles and stabilizer particles is subjected to a pulverization process.

By providing excess energy in the pulverization process under predetermined conditions compared to conventional techniques, the resulting zirconia composition can comprise zirconia particles along with small particles having a stabilizer concentration adjusted within the desired range.

Zirconia itself is a material with notably high hardness. Accordingly, increasing the energy applied in a pulverization process does not readily make the zirconia particles excessively small. From this perspective, excessive pulverization has not been considered to have technical significance.

However, by subjecting the zirconia composition comprising zirconia and yttria to a predetermined pulverization process, it appears possible to adjust the stabilizer concentration in the small particles within the desired range. This is considered to underlie the ability of the present invention to achieve excellent mechanical strength while allowing the opalescence to be controlled within an appropriate range in the sintered body produced, allowing it to exhibit superior aesthetics.

In view of providing excess energy to the zirconia composition and adjusting the stabilizer concentration in the small particles within the desired range to produce a sintered body having excellent mechanical strength while achieving superior aesthetics with the controlled opalescence within an appropriate range, the pulverization process specifically employs a pulverization medium of less than 1 mm in diameter.

As disintegration and grinding progress through the pulverization process, the zirconia and yttria particles become finer while being mixed, allowing for adjustment of the stabilizer concentration in the small particles of the resulting zirconia composition within the desired range.

Moreover, during the pulverization process, yttria progressively forms a solid solution and turns amorphous, allowing the adjustment of the abundance of undissolved yttria according to formula (1-1). The abundance of undissolved yttria is not particularly limited because the zirconia composition, as long as it comprises zirconia particles along with small particles having a stabilizer concentration adjusted within the desired range, can yield a sintered body having excellent mechanical strength and with its opalescence controlled within an appropriate range. The abundance of undissolved yttria can be controlled by adjusting the pulverization time.

Pulverization media with fine diameters help increase the energy level in the pulverization process and adjust the stabilizer concentration in the small particles within the desired range. From this viewpoint, when using a pulverization medium of less than 1 mm in diameter, the size (diameter) of the pulverization medium is preferably 0.1 to 0.5 mm.

The pulverization medium of less than 1 mm in diameter may be a commercially available product.

The pulverization medium of less than 1 mm in diameter may be used with a pulverization device, for example, such as a bead mill.

When using a pulverization medium of less than 1 mm in diameter, the duration of the pulverization process is not particularly limited, as long as the zirconia composition produced comprises zirconia particles with a zirconia concentration of 80 mass% or more, and small particles with a stabilizer concentration of 10 to 50 mass%. However, in view of more readily adjusting the stabilizer concentration in the small particles within the desired range, the pulverization process is preferably 10 minutes or more, more preferably 15 minutes or more, even more preferably 20 minutes or more, particularly preferably 30 minutes or more. In view of more readily adjusting the stabilizer concentration in the small particles within the desired range, the pulverization process is preferably 20 hours or less, more preferably 10 hours or less, even more preferably 5 hours or less, particularly preferably 2 hours or less.

When the pulverization device is a device that operates by circulating slurry (for example, a circulation bead mill), the duration of the pulverization process refers to the period for which the slurry remains in the vessel (pulverization chamber).

In the present invention, by applying increased pulverization energy adjusted through the combination of pulverization time and pulverization medium size, the resulting zirconia composition can comprise zirconia particles along with small particles having a stabilizer concentration adjusted within the desired range.

By using the zirconia composition obtained in the manner described above, which comprises zirconia particles along with small particles having a stabilizer concentration adjusted within the desired range, it is expected that the zirconia sintered body, when produced from the zirconia composition via a zirconia pre-sintered body, can exhibit excellent mechanical strength along with superior aesthetics due to the controlled opalescence within an appropriate range.

In view of achieving excellent mechanical strength and more readily controlling the opalescence within an appropriate range, the stabilizer concentration in the small particles contained in the zirconia composition after pulverization is preferably 10 mass% or more, more preferably 10.5 mass% or more, even more preferably 11 mass% or more, particularly preferably 11.5 mass% or more. In order to achieve excellent mechanical strength and more readily control the opalescence within an appropriate range, the stabilizer concentration in the small particles is preferably 50 mass% or less, more preferably 45 mass% or less, even more preferably 40 mass% or less, particularly preferably 35 mass% or less.

The zirconia composition, after pulverization, may form a group of small particles comprising a plurality of small particles with the adjusted stabilizer concentration within the desired range, provided that it allows the opalescence to be controlled within an appropriate range.

In order to achieve excellent mechanical strength and more readily control the opalescence within an appropriate range, the stabilizer concentration in such group of small particles is preferably 10 to 50 mass%.

Preferably, the stabilizer concentration in the group of small particles is the same or similar to that in the small particles contained in the zirconia composition.

In view of achieving excellent mechanical strength and more readily controlling the opalescence within an appropriate range together with the stabilizer concentration in the small particles, the zirconia concentration in the group of small particles is preferably 50 mass% or more, more preferably 55 mass% or more, even more preferably 60 mass% or more, particularly preferably 65 mass% or more. In view of achieving excellent mechanical strength and more readily controlling the opalescence within an appropriate range together with the stabilizer concentration in the small particles, the zirconia concentration in the group of small particles is preferably 90 mass% or less, more preferably 89.5 mass% or less, even more preferably 89 mass% or less, particularly preferably 88.5 mass% or less.

In view of achieving excellent mechanical strength and more readily controlling the opalescence within an appropriate range, it is preferable in the zirconia composition after pulverization that six or more of ten arbitrarily selected small particles observed in one field magnified 200,000 times under a transmission electron microscope (TEM) are small particles with a stabilizer concentration of 10 to 50 mass%.

In these ten small particles, the number of small particles with a stabilizer concentration of 10 to 50 mass% is more preferably seven or more, even more preferably eight or more, particularly preferably nine or more, most preferably ten.

If ten small particles cannot be observed in one field, an additional field or fields may be used to select a total of ten small particles.

Likewise, in this specification, if the required number of particles cannot be observed in one field when selecting a specific number of small particles (for example, 10) from one field, an additional field or fields may be used to select the required total number of particles (for example, 10). For example, when selecting one hundred particles, ten arbitrary particles may be selected from four fields and the procedure may be repeated until the total reaches the required one hundred particles.

When ten arbitrary small particles are selected and observed across ten fields under TEM, the zirconia composition satisfies the specified number of small particles in preferably six or more fields, more preferably eight or more fields, even more preferably nine or more fields, particularly preferably all ten fields.

In a certain preferred embodiment, the number of small particles with a stabilizer concentration of 10 to 50 mass% in the zirconia composition is preferably sixty or more, more preferably seventy or more, even more preferably eighty or more, particularly preferably ninety or more, most preferably one hundred out of a total of one hundred particles (selected by, for example, choosing ten arbitrary particles per field at 200,000× magnification across ten fields).

In the present invention, the number of particles observed under TEM may be increased to 200 or more, or 500 or more, as necessary.

In view of achieving excellent mechanical strength and more readily controlling the opalescence within an appropriate range, the area ratio (%) of zirconia particles to small particles under observation by TEM is preferably 55/45 to 99/1, more preferably 65/35 to 98/2, even more preferably 70/30 to 97/3, particularly preferably 75/25 to 95/5 in the zirconia composition after pulverization.

The zirconia composition may have various forms, including granules and slurry, both before and after pulverization.

When the zirconia composition is a slurry, it can be produced by mixing the mixed powder from the pulverization process with a solvent (preferably water).

The zirconia composition may additionally comprise additives such as binders, dispersants, emulsifiers, antifoaming agents, pH adjusters, lubricants, and translucency adjusters.

The additives may be used alone, or two or more thereof may be used in combination.

Examples of the binders include polyvinyl alcohol, methyl cellulose, carboxymethyl cellulose, acrylic binders, wax binders, polyvinyl butyral, polymethyl methacrylate, and ethyl cellulose.

For improved translucency, the binder content in a zirconia composition of the present invention is preferably 10 mass% or less, more preferably 5 mass% or less, even more preferably 3 mass% or less relative to 100 mass% of zirconia.

Examples of the plasticizers include polyethylene glycol, glycerin, propylene glycol, and dibutyl phthalate.

Examples of the dispersants include ammonium polycarboxylate (such as triammonium citrate), ammonium polyacrylate, acryl copolymer resins, acrylic acid ester copolymers, polyacrylic acid, bentonite, carboxymethyl cellulose, anionic surfactants (for example, polyoxyethylene alkyl ether phosphoric acid esters such as polyoxyethylene lauryl ether phosphoric acid ester), non-ionic surfactants, oleic glyceride, amine salt surfactants, oligosaccharide alcohols, and stearic acid.

Examples of the emulsifiers include alkyl ethers, phenyl ethers, and sorbitan derivatives.

Examples of the antifoaming agents include alcohols, polyethers, polyethylene glycols, silicone, and waxes.

Examples of the pH adjusters include ammonia, ammonium salts (including ammonium hydroxides such as tetramethylammonium hydroxide).

Examples of the lubricants include polyoxyethylene alkyl ethers, and waxes.

Examples of the translucency adjusters include aluminum oxide ($Al_2O_3$), titanium oxide ($TiO_2$), silicon dioxide ($SiO_2$), zircon, lithium silicate, and lithium disilicate.

The particles constituting the zirconia composition have a BET specific surface area of preferably 7.0 $m^2$/g or more, more preferably 7.5 $m^2$/g or more, even more preferably 8.0 $m^2$/g or more as measured in compliance with JIS Z 8830:2013. When the BET specific surface area is 7.0 $m^2$/g or more, it is easier to inhibit opacification of the sintered body upon sintering. The BET specific surface area is preferably 50 $m^2$/g or less, more preferably 45 $m^2$/g or less, even more preferably 40 $m^2$/g or less. A BET specific surface area of 50 $m^2$/g or less reduces susceptibility to the influence of temperature variations inside the furnace.

The BET specific surface area can be measured using a commercially available product, such as a full automatic specific surface area analyzer (manufactured by Mountech Co., Ltd. under the trade name Macsorb® HM model-1200; BET flow method (single point / multi point)). For example, measurement may be conducted using this full automatic specific surface area analyzer following the BET flow method (single point).

The translucency of the sintered body also becomes less likely to decrease with a shorter firing period in sintering. Here, a "BET specific surface area" is a specific surface area measured without distinguishing primary particles and secondary particles.

In view of providing a sintered body having excellent mechanical strength and with its opalescence controlled within an appropriate range, the average particle diameter of the zirconia composition after pulverization is preferably 0.2 $\mu$m or less.

The average particle diameter of zirconia particles and yttria particles in the zirconia composition after pulverization can be measured using a dynamic light scattering particle size distribution measurement method. For example, measurement may be conducted using a dynamic light scattering particle size distribution analyzer (manufactured by Horiba Ltd. under the trade name SZ-100V2), where a slurry diluted with water to about 0.1 mass% is subjected to ultrasonic waves for 30 minutes, and subsequently measured by volume under continued ultrasonic radiation.

The average primary particle diameter of zirconia particles contained in the zirconia composition after pulverization is preferably 0.01 $\mu$m or more, more preferably 0.02 $\mu$m or more, even more preferably 0.06 $\mu$m or more. The average primary particle diameter of zirconia particles is preferably 0.20 $\mu$m or less, more preferably 0.19 $\mu$m or less, even more preferably 0.17 $\mu$m or less.

The average primary particle diameter of small particles contained in the zirconia composition after pulverization is preferably less than 0.06 $\mu$m, more preferably 0.05 $\mu$m or less, even more preferably 0.04 $\mu$m or less.

The next step is the molding process, where the pulverized zirconia composition is molded to prepare a molded body. The molded body can be obtained by molding the zirconia composition under applied external force using a known method.

The molding method is not particularly limited, and may employ the following methods, for example.

(a) slip casting of a slurry containing the pulverized zirconia composition
(b) gel casting of a slurry containing the pulverized zirconia composition
(c) press molding of the pulverized zirconia composition
(d) molding of a zirconia composition containing zirconia particles, small particles, and resin
(e) polymerization of a zirconia composition containing zirconia particles, small particles, and a polymerizable monomer or oligomer
(f) additive fabrication of granules containing zirconia particles and small particles

(a) Slip Casting

When the zirconia molded body is produced using a method that includes the step of slip casting a slurry containing the pulverized zirconia composition, the slip casting is not particularly limited to specific methods, and may be carried out using, for example, a method that dries the slurry after pouring it in a mold.

For considerations such as ease of pouring the slurry into a mold, and increasing the reusability of the mold in addition to preventing excessive drying time, the content of the dispersion medium in the slurry used is preferably 80 mass% or less, more preferably 50 mass% or less, even more preferably 20 mass% or less.

The slurry may be poured into a mold under ordinary pressure. However, in view of production efficiency, it is preferable to pour the slurry under applied pressure conditions.

(b) Gel Casting

When the zirconia molded body is produced using a method that includes the step of gel casting a slurry containing the pulverized zirconia composition, the gel casting is not particularly limited to specific methods, and may be carried out using, for example, a method that dries a moist body shaped in a mold by gelling the slurry.

For considerations such as preventing excessive drying time and reducing cracking during drying, the content of the dispersion medium contained in the slurry used is preferably 80 mass% or less, more preferably 50 mass% or less, even more preferably 20 mass% or less.

The gelation may be achieved by, for example, adding a gelatinizer, or by adding and polymerizing a polymerizable monomer.

The gelatinizer is not particularly limited, and may be, for example, a water-soluble gelatinizer. Specifically, preferred are agarose and gelatin, for example. The gelatinizer may be used alone, or two or more thereof may be used in combination. The gelatinizer may be used in any amount, as long as it does not cause problems such as cracking during sintering. The gelatinizer may be used in an amount of 10 mass% or less, 5 mass% or less, or 1 mass% or less, based on the mass the slurry after the addition of gelatinizer.

The polymerizable monomer is not particularly limited, and may be, for example, a (meth)acrylate monomer such as 2-hydroxyethyl (meth)acrylate, 3-hydroxypropyl (meth)acrylate, 4-hydroxybutyl (meth)acrylate, 6-hydroxyhexyl (meth) acrylate, 10-hydroxydecyl (meth)acrylate, propylene glycol mono(meth)acrylate, glycerol mono(meth)acrylate, or erythritol mono(meth)acrylate; or a (meth)acrylamide monomer such as N-methylol(meth)acrylamide, N-hydroxyethyl(meth) acrylamide, or N,N-bis(2-hydroxyethyl)(meth)acrylamide.

The polymerizable monomer may be used alone, or two or more thereof may be used in combination.

The polymerizable monomer may be used in any amount, as long as it does not cause problems such as cracking during sintering. The polymerizable monomer may be used in an amount of 10 mass% or less, 5 mass% or less, or 1 mass% or less, based on the mass the slurry after the addition of polymerizable monomer.

When gelation is performed through polymerization of a polymerizable monomer, it is preferable to use a polymerization initiator for polymerization. The polymerization initiator is not particularly limited. Particularly preferred are photopolymerization initiators. The photopolymerization initiator can be appropriately selected from photopolymerization initiators used in industry, preferably those used in dentistry.

Specific examples of photopolymerization initiators include (bis)acylphosphine oxides (including salts), thioxanthones (including salts such as quaternary ammonium salts), ketals, $\alpha$-diketones, coumarins, anthraquinones, benzoin alkyl ether compounds, and $\alpha$-aminoketone compounds. The photopolymerization initiators may be used alone, or two or more thereof may be used in combination. Preferred among these photopolymerization initiators is at least one selected from the group consisting of (bis)acylphosphine oxides and $\alpha$-diketones. With these photopolymerization initiators, it is possible to achieve polymerization (gelation) both in the ultraviolet region (including the near ultraviolet region) and the visible region. Specifically, polymerization (gelation) can sufficiently take place irrespective of whether the light source used is a laser such as an Ar laser or a He-Cd laser, or a lamp such as a halogen lamp, a xenon lamp, a metal halide lamp, a light emitting diode (LED), a mercury lamp, or a fluorescent lamp.

The method used to dry the shaped moist body is not particularly limited, and may be, for example, natural drying, hot-air drying, vacuum drying, drying by dielectric heating, drying by induction heating, or drying under constant temperature and humidity. These methods may be used alone, or two or more thereof may be used in combination. In view of considerations such as the capability to reduce cracking during drying, preferred are natural drying, drying by dielectric heating, drying by induction heating, and drying under constant temperature and humidity.

The molds used for the slip casting and gel casting are not particularly limited, and may be, for example, porous molds made of a material such as plaster, resin, or ceramic, or non-porous molds made of a material such as metal or resin.

(c) Press Molding

In the press molding of the pulverized zirconia composition, the press molding is not particularly limited to specific methods, and may be carried out using a known press molding machine.

Specific examples of the press molding method include uniaxial pressing.

The press molding may be conducted in multiple stages. For example, the zirconia composition after press molding may be subjected to CIP (Cold Isostatic Pressing).

The shape of the molded body is not particularly limited, and may be disk-shaped, cuboidal, or the form of a dental product (for example, a crown shape).

The molded body may be, for example, a columnar zirconia molded body formed by filling a mold with the zirconia composition (for example, granules) and compacting it by uniaxial pressing.

The density of the molded body increases with higher surface pressure in press molding. However, the zirconia molded body yields a hard zirconia pre-sintered body when its density is too high. Accordingly, the surface pressure of press molding for the preparation of the zirconia molded body is preferably 30 to 200 MPa. When the surface pressure is 30 MPa or more, superior shape retention can be achieved in the zirconia molded body, whereas a surface pressure of 200 MPa or less prevents the density of the zirconia molded body from becoming too high and helps avoid excessive hardening more effectively.

The molded body includes those densified through high-temperature pressing such as CIP (Cold Isostatic Pressing).

From the same perspectives described above, the CIP pressure is preferably 30 to 200 MPa.

(d) Molding of Zirconia Composition Containing Resin

When the zirconia molded body is produced using a method that comprises the step of molding a zirconia composition containing zirconia particles, small particles, and resin, the method used to mold the zirconia composition is not particularly limited to specific methods, and may be, for example, injection molding, cast molding, or extrusion molding.

It is also possible to use additive fabrication techniques (such as 3D printing) to form the composition, including, for example, fused deposition modeling (FDM), the inkjet method, and the powder/binder lamination method. Preferred among these molding methods are injection molding and cast molding, more preferably injection molding.

The resin is not particularly limited, and is preferably the binder described above.

(e) Polymerization of Zirconia Composition Containing Zirconia Particles, Small Particles, and Polymerizable Monomer or Oligomer

By polymerizing a zirconia composition containing zirconia particles, small particles, and a polymerizable monomer or oligomer, the polymerizable monomer or oligomer in the zirconia composition can polymerize, allowing the composition to cure.

When the zirconia molded body is produced using a method that comprises such a polymerization step, the method is not particularly limited to specific methods, and may be, for example, a method by which a zirconia composition is polymerized in a mold, or stereolithography (SLA) using a zirconia composition. The preferred method is stereolithography (SLA).

Stereolithography enables the zirconia molded body to have a shape corresponding to the shape desired for the final zirconia sintered body, at the time of its production. This makes stereolithography a potentially preferred method in certain situations, particularly when the zirconia sintered body is used as a dental material such as a dental prosthesis.

The polymerizable monomer is not particularly limited, and may be a monofunctional polymerizable monomer such as a monofunctional (meth)acrylate or a monofunctional (meth)acrylamide, or a polyfunctional polymerizable monomer such as a bifunctional aromatic compound, a bifunctional aliphatic compound, or a tri- or higher-functional compound. The polymerizable monomer may be used alone, or two or more thereof may be used in combination. Preferred for use are polyfunctional polymerizable monomers, particularly in situations where stereolithography is employed.

The oligomer is not particularly limited, as long as it is a polymerizable compound formed by the bonding of two or more of the aforementioned polymerizable monomers.

Examples of the monofunctional (meth)acrylate include:

(meth)acrylates having a hydroxyl group(s), such as 2-hydroxyethyl (meth)acrylate, 2-hydroxypropyl (meth)acrylate, 3-hydroxypropyl (meth)acrylate, 4-hydroxybutyl (meth)acrylate, 6-hydroxyhexyl (meth)acrylate, 10-hydroxydecyl (meth)acrylate, propylene glycol mono(meth)acrylate, glycerol mono(meth)acrylate, and erythritol mono(meth) acrylate;

alkyl (meth)acrylates such as methyl (meth)acrylate, ethyl (meth)acrylate, propyl (meth)acrylate, isopropyl (meth) acrylate, n-butyl (meth)acrylate, sec-butyl (meth)acrylate, t-butyl (meth)acrylate, isobutyl (meth)acrylate, n-hexyl (meth)acrylate, lauryl (meth)acrylate, cetyl (meth)acrylate, and stearyl (meth)acrylate;

alicyclic (meth)acrylates such as cyclohexyl (meth)acrylate, and isobornyl (meth)acrylate;

aromatic group-containing (meth)acrylates such as benzyl (meth)acrylate, and phenyl (meth)acrylate; and

(meth)acrylates having functional groups, such as 2,3-dibromopropyl (meth)acrylate, 3-(meth)acryloyloxypropyltrimethoxysilane, and 11-(meth)acryloyloxyundecyltrimethoxysilane.

Examples of the monofunctional (meth)acrylamide include (meth)acrylamide, N-(meth)acryloylmorpholine, N,N-dimethyl(meth)acrylamide, N,N-diethyl(meth)acrylamide, N,N-di-n-propyl(meth)acrylamide, N,N-di-n-butyl(meth) acrylamide, N,N-di-n-hexyl(meth)acrylamide, N,N-di-n-octyl(meth)acrylamide, N,N-di-2-ethylhexyl(meth)acrylamide, N-hydroxyethyl(meth)acrylamide, and N,N-bis(2-hydroxyethyl)(meth)acrylamide.

In view of excellent polymerizability, preferred among these monofunctional polymerizable monomers are (meth) acrylamides, more preferably N-(meth)acryloylmorpholine, N,N-dimethyl(meth)acrylamide, and N,N-diethyl(meth)acrylamide.

Examples of the bifunctional aromatic compound include (meth)acrylates such as 2,2-bis((meth)acryloyloxyphenyl)propane, 2,2-bis[4-(2-hydroxy-3-acryloyloxy-2-hydroxypropoxy)phenyl]propane, 2,2-bis[4-(2-hydroxy-3-methacryloyloxypropoxy)phenyl]propane (commonly known as Bis-GMA), 2,2-bis(4-(meth)acryloyloxyethoxyphenyl) propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, and 1,4-bis(2-(meth)acryloyloxyethyl) pyromellitate. In view of excellent polymerizability and providing a zirconia molded body having superior mechanical strength, preferred are Bis-GMA, and 2,2-bis(4-(meth)acryloyloxypolyethoxyphenyl)propane. Preferred as 2,2-bis(4-(meth)acryloyloxypolyethoxyphenyl)propane is 2,2-bis(4-methacryloyloxypolyethoxyphenyl)propane (with an average of 2.6 moles of ethoxy group added; commonly known as D-2.6E).

Examples of the bifunctional aliphatic compound include (meth)acrylates such as glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,4-butanediol di(meth)acrylate, 1,6-hexanediol di(meth)acrylate, 2-ethyl-1,6-hexanediol di(meth)acrylate, 1,9-nonanediol di(meth)acrylate, 1,10-decanediol di(meth)acrylate, 1,2-bis(3-methacryloyloxy-2-hydroxypropoxy)ethane, and 2,2,4-trimethylhexamethylenebis(2-carbamoyloxyethyl)dimethacrylate (commonly known as UDMA). In view of excellent polymerizability and providing a zirconia molded body having superior mechanical strength, preferred are triethylene glycol dimethacrylate (commonly known as TEGDMA), and UDMA.

Examples of the tri- or higher-functional compound include (meth)acrylates such as trimethylolpropane tri(meth) acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis [2-(aminocarboxy)propane-1,3-diol]tetra(meth)acrylate, and 1,7-diacryloyloxy-2,2,6,6-tetra(meth)acryloyloxymethyl-4-oxaheptane. In view of excellent polymerizability and providing a zirconia molded body having superior mechanical strength, preferred are N,N-(2,2,4-trimethylhexamethylene)bis[2-(aminocarboxy)propane-1,3-diol]tetramethacrylate, and 1,7-diacryloyloxy-2,2,6,6-tetraacryloyloxymethyl-4-oxaheptane.

In both of the foregoing methods, it is preferable that the composition be polymerized using a polymerization initiator, and that the composition additionally comprise a polymerization initiator. The polymerization initiator is not particularly limited. Particularly preferred are photopolymerization initiators. The photopolymerization initiator may be appropriately selected from photopolymerization initiators used in industry, preferably those used in dentistry. Specific examples of the photopolymerization initiator are as indicated above in the description of gel casting.

When the zirconia molded body is produced by stereolithography using the zirconia composition after pulverization, the stereolithography method is not particularly limited to specific methods, and a known method may be appropriately selected for stereolithography. For example, it is possible to use a method that produces the desired zirconia molded body by successively forming layers in desired shapes through photopolymerization of a liquid composition with, for example, ultraviolet light or a laser, using a stereolithography device.

The zirconia molded body, as the cured product, may be subjected to a CIP process after a humidification process, in order to further improve the density of the zirconia molded body.

When press molding is performed, the zirconia particle-containing powder may be subjected to press molding after a humidification process, before pressing the powder. Any known methods can be used for the humidification process, including a method that sprays water with a sprayer or the like, and a method that uses a constant humidity chamber or a constant temperature and humidity chamber. The amount of moisture increase after the humidification process is preferably more than 2 mass%, more preferably more than 3 mass%, even more preferably more than 4 mass%, particularly preferably more than 5 mass%, and is preferably 15 mass% or less, more preferably 13 mass% or less, even more preferably 11 mass% or less relative to the mass of the unwetted powder (powder before humidification process) and molded body, though the amount of moisture increase depends on factors such as the average particle size of the zirconia particles and stabilizer particles contained. The amount of moisture increase by humidification process can be determined as a percentage by subtracting the mass of the unwetted powder and molded body from the mass of the wetted powder (powder after humidification process) and molded body, and dividing the difference by the mass of the unwetted powder and molded body.

The CIP pressure is as indicated above in the description of press molding.

(f) Additive Fabrication of Granules Containing Zirconia Particles and Small Particles

There is no specific restriction on the method for producing granules containing zirconia particles and small particles. For example, granules can be obtained by preparing a slurry and drying it with a spray dryer, and these granules may be used for powder additive fabrication.

The technique employed for powder additive fabrication is not particularly limited. Examples include the powder bed method, SLS method (selective laser sintering), SLM method (selective laser melting), electron beam method, arc discharge method, and binder jet method. For techniques where organic materials should be excluded in additive fabrication, it is preferable to also avoid using organic materials during the production of granules.

[Production Method of Zirconia Pre-Sintered Body]

The following describes a method for producing the zirconia pre-sintered body through the case where the stabilizer is yttria.

A zirconia pre-sintered body of the present invention can be produced by pulverizing the zirconia composition, and firing (pre-sintering) a molded body of the pulverized zirconia composition to an extent that the zirconia particles do not sinter, with the pulverization process providing excess energy under predetermined conditions compared to conventional techniques.

In view of ensuring a semi-sintered state using the specific zirconia composition above, the pre-sintering temperature (highest pre-sintering temperature) in the pre-sintering step is preferably 600°C or more, more preferably 700°C or more, even more preferably 800°C or more, particularly preferably 850°C or more.

In view of ensuring processability, the pre-sintering temperature is preferably 1,200°C or less, more preferably 1,150°C or less, even more preferably 1,100°C or less, particularly preferably 1,050°C or less.

That is, the preferred pre-sintering temperature is 600°C to 1,200°C in the method of production of a zirconia pre-sintered body of the present invention.

With the pulverization process yielding small particles with the stabilizer concentration confined within the predetermined range, it is possible to obtain the desired pre-sintered body even at temperatures (for example, about 600 to 900°C) lower than those conventionally employed.

The hold time (retention time) at the highest pre-sintering temperature is not particularly limited, as long as it can yield a zirconia pre-sintered body in a semi-sintered state. Preferably, the highest pre-sintering temperature is held for 30 minutes to 6 hours.

The rate of temperature increase to the highest pre-sintering temperature and the rate of temperature decrease from the highest pre-sintering temperature are preferably 300°C/min or less.

In certain preferred embodiments, the rate of temperature increase to the highest pre-sintering temperature in pre-sintering of a zirconia molded body of the present invention is not particularly limited. However, the rate of temperature increase to the highest pre-sintering temperature is preferably 0.1°C/min or more, more preferably 0.2°C/min or more, even more preferably 0.5°C/min or more, and is preferably 50°C/min or less, more preferably 30°C/min or less, even more preferably 20°C/min or less.

The productivity improves when the rate of temperature increase is at or above these lower limits. When the rate of temperature increase is at or below the foregoing upper limits, it is possible to reduce the volume difference between the inner and outer portions of the zirconia molded body and zirconia pre-sintered body. Additionally, when the zirconia molded body contains organic materials, the rapid decomposition of these organic materials can be reduced to inhibit cracks or fractures.

[Zirconia Pre-Sintered Body]

The following describes the zirconia pre-sintered body.
The zirconia pre-sintered body is obtained by pre-sintering the zirconia molded body obtained in the manner described above.
The type and content of the stabilizer in the zirconia pre-sintered body are as indicated above for the zirconia composition.
There is no particular restriction on a zirconia pre-sintered body of the present invention because a zirconia sintered body having excellent mechanical strength and with its opalescence controlled within an appropriate range can be obtained with the use of the zirconia composition. However, in view of producing a sintered body with potentially higher mechanical strength and translucency in combination with the monoclinic fraction of zirconia being in a predetermined range, it is preferable in a certain embodiment of a zirconia pre-sintered body of the present invention that the abundance $f_y$ (%) of yttria not dissolved in zirconia as a solid solution (hereinafter, also referred to simply as "undissolved yttria") is less than 4% as calculated by the following formula (1-1).

$$f_y\ (\%) = I_y / (I_m + I_t + I_c + I_y) \times 100 \qquad (1\text{-}1),$$

wherein $f_y$ represents the abundance (%) of yttria not dissolved in zirconia as a solid solution, $I_m$ represents the integrated intensity of the peak near $2\theta = 28.2°$, where the peak top of the main peak of the monoclinic zirconia appears in XRD measurement, It represents the integrated intensity of the peak near $2\theta = 30.3°$, where the peak top of the main peak of the tetragonal zirconia appears in XRD measurement, $I_c$ represents the integrated intensity of the peak near $2\theta = 30.0°$, where the peak top of the main peak of the cubic zirconia appears in XRD measurement, and $I_y$ represents the integrated intensity of the peak near $2\theta = 29.2°$, where the peak top of the main peak of yttria not dissolved in zirconia as a solid solution appears in XRD measurement.
Given that the main peaks of the tetragonal zirconia and cubic zirconia occur at similar positions, the integrated intensity of each main peak is calculated through peak separation.
In view of achieving even superior mechanical strength and translucency in the zirconia sintered body through the combined effect of the width at half maximum being in a predetermined range, the abundance $f_y$ (%) of undissolved yttria is preferably less than 3.5%, more preferably 2% or less, particularly preferably 1% or less.
The abundance $f_y$ (%) of undissolved yttria may be 0%.
The abundance $f_y$ (%) of undissolved yttria can be adjusted through processes such as pulverization, as will be described later.
There is no particular restriction on a zirconia pre-sintered body of the present invention because a zirconia sintered body having excellent mechanical strength and with its opalescence controlled within an appropriate range can be obtained with the use of the zirconia composition. However, in view of producing a sintered body with potentially higher mechanical strength and translucency in combination with the small particles with the adjusted stabilizer concentration within the desired range, it is preferable in a certain embodiment of a zirconia pre-sintered body of the present invention that the monoclinic fraction $f_m$ (%) is less than 55% as calculated by formula (1-2).

$$f_m\ (\%) = I_m / (I_m + I_t + I_c + I_y) \times 100 \qquad (1\text{-}2),$$

wherein $f_m$ represents the monoclinic fraction (%), $I_m$ represents the integrated intensity of the peak near $2\theta = 28.2°$, where the peak top of the main peak of the monoclinic zirconia appears in XRD measurement, It represents the integrated intensity of the peak near $2\theta = 30.3°$, where the peak top of the main peak of the tetragonal zirconia appears in XRD measurement, $I_c$ represents the integrated intensity of the peak near $2\theta = 30.0°$, where the peak top of the main peak of the cubic zirconia appears in XRD measurement, and $I_y$ represents the integrated intensity of the peak near $2\theta = 29.2°$, where the peak top of the main peak of undissolved yttria appears in XRD measurement.
Given that the main peaks of the tetragonal zirconia and cubic zirconia occur at similar positions, the integrated intensity of each main peak is calculated through peak separation.
In view of achieving even superior mechanical strength and translucency in the zirconia sintered body through the combined effect of the width at half maximum being in a predetermined range, the monoclinic fraction $f_m$ (%) is more

preferably less than 50%, even more preferably 48% or less, particularly preferably 45% or less.

The monoclinic fraction $f_m$ (%) is preferably 5% or more, more preferably 10% or more, even more preferably 15% or more, particularly preferably 20% or more.

A zirconia pre-sintered body of the present invention may comprise additives other than zirconia and stabilizers, provided that the present invention can exhibit its effects. Examples of such additives include colorants (including pigments, composite pigments, and fluorescent agents), alumina ($Al_2O_3$), titanium oxide ($TiO_2$), and silica ($SiO_2$).

Examples of the pigments include oxides of at least one element selected from the group consisting of Ti, V, Cr, Mn, Fe, Co, Ni, Zn, Y, Sn, Sb, Bi, Ce, Pr, Sm, Eu, Gd, Tb, and Er (specifically, such as NiO and $Cr_2O_3$), excluding $Y_2O_3$ and $CeO_2$, preferably oxides of at least one element selected from the group consisting of Ti, V, Cr, Mn, Fe, Co, Ni, Zn, Y, Sn, Sb, Bi, Ce, Pr, Sm, Eu, Gd, and Tb, more preferably oxides of at least one element selected from the group consisting of Ti, V, Cr, Mn, Fe, Co, Ni, Zn, Y, Sn, Sb, Bi, Ce, Sm, Eu, Gd, and Tb.

A zirconia pre-sintered body of the present invention may be one that does not comprise erbium oxide ($Er_2O_3$).

Examples of the composite pigments include $(Zr,V)O_2$, $Fe(Fe,Cr)_2O_4$, $(Ni,Co,Fe)(Fe,Cr)_2O_4 \cdot ZrSiO_4$, and $(Co,Zn)Al_2O_4$. Examples of the fluorescent agents include $Y_2SiO_5$:Ce, $Y_2SiO_5$:Tb, $(Y,Gd,Eu)BO_3$, $Y_2O_3$:Eu, YAG:Ce, $ZnGa_2O_4$:Zn, and $BaMgAl_{10}O_{17}$:Eu.

A zirconia pre-sintered body of the present invention may comprise a fluorescent agent. The zirconia sintered body can exhibit fluorescence by incorporating a fluorescent agent in the zirconia pre-sintered body. The type of fluorescent agent is not particularly limited, and it is possible to use a single fluorescent agent or two or more fluorescent agents capable of emitting fluorescence at any wavelength of light.

Examples of the fluorescent agent include those containing metal elements. Examples of the metal elements include Ga, Bi, Ce, Nd, Sm, Eu, Gd, Tb, Dy, and Tm. The fluorescent agent may contain one of these metal elements by itself, or may contain two or more of these metal elements. Preferred among these metal elements are Ga, Bi, Eu, Gd, and Tm, more preferably Bi and Eu.

Examples of such fluorescent agents include oxides, hydroxides, acetates, and nitrates of the metal elements above. The fluorescent agent may be, for example, $Y_2SiO_5$:Ce, $Y_2SiO_5$:Tb, $(Y,Gd,Eu)BO_3$, $Y_2O_3$:Eu, YAG:Ce, $ZnGa_2O_4$:Zn, or $BaMgAl_{10}O_{17}$:Eu.

The content of the fluorescent agent in the zirconia pre-sintered body is not particularly limited, and may be appropriately adjusted according to factors such as the type of fluorescent agent, and the intended use of the zirconia sintered body. However, in view of considerations such as suitability as dental prostheses, the content of fluorescent agent is preferably 0.001 mass% or more, more preferably 0.005 mass% or more, even more preferably 0.01 mass% or more in terms of the oxide equivalent of the metal elements contained in the fluorescent agent, relative to 100 mass% of the zirconia contained in the zirconia pre-sintered body.

The content of fluorescent agent is not particularly limited, and may be 1 mass% or less, 0.5 mass% or less, or 0.1 mass% or less in terms of the oxide equivalent of the metal elements contained in the fluorescent agent, provided that the fluorescent agent can exhibit suitable fluorescence. When the content is at or above these lower limits, the fluorescence can match or exceed that of natural human teeth. When the content is at or below the foregoing upper limits, it is possible to reduce a decrease in mechanical strength and translucency.

In order to ensure strength that enables mechanical processing in a pre-sintered state, the flexural strength of a zirconia pre-sintered body of the present invention is preferably 15 MPa or more. For ease of mechanical processing in a pre-sintered state, the flexural strength of the zirconia pre-sintered body is preferably 70 MPa or less, more preferably 60 MPa or less.

The flexural strength can be measured in compliance with ISO 6872:2015 (Dentistry-Ceramic materials). For measurement, specimens measuring 5 mm × 10 mm × 50 mm are used under the same conditions except for size. For surface finishing, the specimen surfaces, including the chamfered surface (45° chamfers at the corners of specimen), are finished longitudinally with #600 sandpaper. The specimen is disposed in such an orientation that its widest face is perpendicular to the vertical direction (loading direction). In the three-point flexural test, measurement is carried out at a crosshead speed of 0.5 mm/min with the distance between supports (span) set at 30 mm.

A zirconia pre-sintered body of the present invention has a density of preferably 2.7 $g/cm^3$ or more, more preferably 3.0 $g/cm^3$ or more, even more preferably 3.2 $g/cm^3$ or more.

The zirconia pre-sintered body has a density of preferably 4.0 $g/cm^3$ or less, more preferably 3.8 $g/cm^3$ or less, even more preferably 3.6 $g/cm^3$ or less. A density in these ranges allows for easy molding.

The density of the zirconia pre-sintered body can be calculated as the mass-to-volume ratio (mass of zirconia pre-sintered body) / (volume of zirconia pre-sintered body). For example, the density of the zirconia pre-sintered body can be determined by cutting out test specimens with dimensions of 10 mm × 10 mm × 10 mm from arbitrary locations of the zirconia pre-sintered body (n = 3), measuring the mass and volume of each test specimen, and averaging the measured values. The calculated arithmetic average can then be applied to the above formula to determine the density.

[Zirconia Sintered Body]

The following describes the zirconia sintered body.

The zirconia sintered body is obtained by firing the zirconia pre-sintered body obtained in the manner described above.

The content of the stabilizer (preferably, yttria) in a zirconia sintered body of the present invention is as indicated above for the zirconia pre-sintered body.

A zirconia sintered body of the present invention uses raw materials including small particles having a stabilizer concentration adjusted within the desired range. This effectively enables the opalescence to be controlled within an appropriate range even in a zirconia sintered body fabricated with a retention time (hold time) of 10 minutes or less at the highest sintering temperature (for example, 1,550°C) in firing.

A zirconia sintered body of the present invention may comprise a fluorescent agent. The fluorescent agent is as described above for the zirconia pre-sintered body.

In this specification, concerning the fluorescent agent, the phrase "relative to 100 mass% of zirconia contained in zirconia pre-sintered body" can be read as "relative to 100 mass% of zirconia contained in zirconia sintered body."

A zirconia sintered body of the present invention may comprise a colorant. Examples of the colorant include those exemplified above for the zirconia pre-sintered body.

The colorant content in the zirconia sintered body is not particularly limited, and may be appropriately adjusted according to factors such as the type of colorant, and the intended use of the zirconia sintered body. However, in view of considerations such as suitability as dental prostheses, the colorant content is preferably 0.001 mass% or more, more preferably 0.005 mass% or more, even more preferably 0.01 mass% or more in terms of the oxide equivalent of the metal elements contained in the colorant, relative to 100 mass% of zirconia contained in the zirconia sintered body. The colorant content is preferably 5 mass% or less, more preferably 1 mass% or less, even more preferably 0.5 mass% or less in terms of the oxide equivalent of the metal elements contained in the colorant. The colorant content may be 0.1 mass% or less, or 0.05 mass% or less.

A zirconia sintered body of the present invention may comprise a translucency adjuster, in order to adjust the translucency of the zirconia sintered body. Examples of the translucency adjuster include those exemplified above for the zirconia pre-sintered body.

The content of the translucency adjuster in the zirconia sintered body is not particularly limited, and may be appropriately adjusted according to factors such as the type of translucency adjuster, and the intended use of the zirconia sintered body. However, in view of considerations such as suitability as dental prostheses, the content of the translucency adjuster is preferably 0.1 mass% or less relative to 100 mass% of zirconia contained in the zirconia sintered body.

[Production Method of Zirconia Sintered Body]

A zirconia sintered body of the present invention can be produced by, for example, a method that fires the zirconia pre-sintered body.

Preferably, the method comprises the step of firing the zirconia pre-sintered body at a highest sintering temperature of more than 1,200°C and 1,650°C or less under ordinary pressure (hereinafter, also referred to as "firing step").

In a certain preferred embodiment, a zirconia pre-sintered body of the present invention, by comprising small particles with the stabilizer concentration adjusted within the desired range, can easily yield a zirconia sintered body of the present invention that exhibits excellent translucency even after short sintering with a hold time of, for example, 10 minutes or less at the highest sintering temperature.

When the sintered body is produced by firing a zirconia pre-sintered body of the present invention, it is preferable to employ highest sintering temperature conditions that maximize the translucency of the zirconia sintered body.

In this connection, in view of considerations such as allowing easy production of the desired zirconia sintered body under ordinary pressure, the highest sintering temperature is preferably more than 1,200°C, more preferably 1,250°C or more, even more preferably 1,300°C or more. The highest sintering temperature is preferably 1,650°C or less, more preferably 1,600°C or less, even more preferably 1,550°C or less, particularly preferably 1,500°C or less, most preferably 1,450°C or less.

A certain embodiment is, for example, a method for producing a zirconia sintered body with a firing step involving a highest sintering temperature of more than 1,200°C and 1,450°C or less.

When the highest sintering temperature is at or above the foregoing lower limits and at or below the upper limits specified above, the sintering process can sufficiently proceed, easily yielding a dense sintered body. When the highest sintering temperature is at or below the foregoing upper limits, it is possible to inhibit deactivation of the fluorescent agent.

The hold time at the highest sintering temperature is preferably 15 minutes or more, more preferably 30 minutes or more, even more preferably 40 minutes or more. The hold time at the highest sintering temperature is preferably 20 hours or less, more preferably 5 hours or less, even more preferably 2 hours or less.

When producing a sintered body by short sintering, the sintering time is not particularly limited, as long as the hold time at the highest sintering temperature is 10 minutes or less. However, in view of considerations such as enabling efficient and stable production of the desired zirconia sintered body with good productivity, the hold time at the highest sintering temperature is preferably 10 minutes or less, more preferably 5 minutes or less, even more preferably 3 minutes or less, particularly preferably 2 minutes or less.

The hold time may be 30 seconds or more, 45 seconds or more, or 1 minute or more.

In producing the sintered body, a shorter firing time can be employed for the fabrication of the sintered body without causing a decrease in the translucency of the zirconia sintered body prepared. Particularly, the hold time at the highest sintering temperature can be reduced to 10 minutes or less for the fabrication of the sintered body. This can lead to enhanced production efficiency, and, when a zirconia pre-sintered body of the present invention is applied to dental products, the patient can experience less time-related stress because the time required from deciding the product dimensions and performing machining to making the dental product ready for treatment can be reduced. It is also possible to reduce the energy cost.

Preferably, the rate of temperature increase and the rate of temperature decrease in the firing process are set so as to reduce the time required for the firing process. For example, the rate of temperature increase may be set so that the highest sintering temperature is reached in the shortest possible time, depending on the capabilities of the furnace. The rate of temperature increase to the highest sintering temperature may be, for example, 10°C/min or more, 50°C/min or more, 100°C/min or more, 120°C/min or more, 150°C/min or more, 200°C/min or more, 250°C/min or more, 300°C/min or more, or 350°C/min or more. Preferably, the rate of temperature decrease is set to a rate that does not cause defects, such as cracks, in the sintered body. For example, the sintered body may be allowed to cool at room temperature after the heating is finished.

In the present invention, a firing furnace can be used for pre-sintering and firing. The type of furnace is not particularly limited, and may be, for example, a furnace used in industry, such as an electric furnace or debinding furnace.

It is also possible to use a commercially available dental firing furnace (for example, Sintra CS manufactured by Shenpaz under this trade name).

A zirconia sintered body of the present invention can be produced even without a HIP process. However, the translucency and mechanical strength can further improve when a HIP process, as an optional process, follows the sintering performed under ordinary pressure.

In the following, the term "primary sintered body" is used to refer to a sintered body obtained through sintering at the highest sintering temperature (sintered body before HIP processing), whereas "HIP sintered body" is used to refer to a sintered body after HIP processing.

Known hot isostatic pressing (HIP) devices can be used for HIP processing.

The HIP pressure in the HIP process of the primary sintered body is not particularly limited. However, for advantages such as obtaining a dense sintered body with high mechanical strength, the HIP pressure is preferably 100 MPa or more, more preferably 125 MPa or more, even more preferably 130 MPa or more. The upper limit of HIP pressure is not particularly limited, and may be, for example, 400 MPa or less, 300 MPa or less, or 200 MPa or less.

The rate of temperature increase in the HIP process of the primary sintered body is not particularly limited, and is preferably 0.1°C/min or more, more preferably 0.2°C/min or more, even more preferably 0.5°C/min or more. The rate of temperature increase is preferably 50°C/min or less, more preferably 30°C/min or less, even more preferably 20°C/min or less. Productivity improves when the rate of temperature increase is at or above the foregoing lower limits.

The duration of HIP in the HIP process of the primary sintered body is not particularly limited. However, for advantages such as obtaining a dense zirconia sintered body with high mechanical strength, the duration of the HIP process is preferably 5 minutes or more, more preferably 10 minutes or more, even more preferably 30 minutes or more. The duration of the HIP process is preferably 10 hours or less, more preferably 6 hours or less, even more preferably 3 hours or less.

In the method for producing a zirconia sintered body of the present invention, the pressure medium in the HIP process of the primary sintered body is not particularly limited. However, in view of a low impact on zirconia, the pressure medium may be at least one selected from the group consisting of oxygen, oxygen with 3% hydrogen, air, and various inert gases (for example, nitrogen, argon).

When conducting HIP processing of the primary sintered body in an oxygen-mixed gas atmosphere, the oxygen concentration may be, for example, more than 0% and 20% or less, though it is not particularly limited.

When using an oxygen-mixed gas, at least one inert gas (for example, such as nitrogen or argon) may be selected as a gas other than oxygen.

The HIP process may result in blackening due to oxygen deficiency when the HIP process is carried out in a reducing atmosphere such as by using an inert gas. In order to prevent such blackening, it is preferable to include a heat treatment step (or tempering as it is also called hereinbelow), performed at 1,650°C or less in either the atmosphere or an excess oxygen atmosphere, after the HIP process. In view of the efficiency of heat treatment, the heat treatment step is more preferably carried out in an excess oxygen atmosphere.

Here, excess oxygen atmosphere means an atmosphere where the oxygen concentration exceeds that of the atmosphere.

The excess oxygen atmosphere is not particularly limited, as long as the oxygen concentration is more than 21% and 100% or less, and can be appropriately selected within this range. For example, the oxygen concentration may be 100%. The HIP process may result in blackening due to oxygen deficiency when the HIP process is carried out in a reducing atmosphere such as by using an inert gas. In order to prevent such blackening, it is preferable to include a heat treatment step (or tempering as it is also called hereinbelow), performed at 1,650°C or less in either the atmosphere or an excess oxygen atmosphere, after the HIP process. In view of the efficiency of heat treatment, the heat treatment step is more preferably carried out in an excess oxygen atmosphere. Here, excess oxygen atmosphere means an atmosphere where the oxygen concentration exceeds that of the atmosphere. The excess oxygen atmosphere is not particularly limited, as long as the oxygen concentration is more than 21% and 100% or less, and can be appropriately selected within this range. For example, the oxygen concentration may be 100%.

A zirconia sintered body of the present invention may be a primary sintered body, a HIP sintered body, or a tempered sintered body with no particular restriction, provided that the present invention can exhibit its effects.

The heat treatment temperature in the atmosphere or an excess oxygen atmosphere may be appropriately modified according the aesthetics of the zirconia sintered body (for example, the shade of dental prostheses).

In a certain preferred embodiment, in view of the aesthetics of the zirconia sintered body, the heat treatment temperature in the atmosphere or an excess oxygen atmosphere is preferably 1,650°C or less, more preferably 1,600°C or less, even more preferably 1,550°C or less.

In another preferred embodiment, in view of the aesthetics of the zirconia sintered body, the heat treatment temperature in the atmosphere or an excess oxygen atmosphere is preferably 1,400°C or less, more preferably 1,300°C or less, even more preferably 1,200°C or less.

In either embodiment, the heat treatment temperature is preferably 500°C or more, more preferably 600°C or more, even more preferably 700°C or more.

A common dental zirconia furnace can be used for the tempering process. The dental zirconia furnace may be a commercially available product. Examples of commercially available products include Noritake KATANA® F-1, F-1N, F-2, F-2N (SK Medical Electronics Co., Ltd.).

The zirconia sintered body produced by firing a zirconia pre-sintered body of the present invention can be suitably used as a dental product.

Examples of such dental products include copings, frameworks, crowns, crown bridges, abutments, implants, implant screws, implant fixtures, implant bridges, implant burs, brackets, denture bases, inlays, onlays, orthodontic wires, and laminate veneers.

Using a zirconia pre-sintered body of the present invention for components such as implant screws and implant fixtures can reduce metal-induced gingival discoloration associated with the use of metallic materials, thereby enhancing aesthetics.

Additionally, a suitable producing method can be chosen depending on the intended application. For example, a dental product can be obtained by firing a zirconia pre-sintered body of the present invention after it has been machined. The use of a CAD/CAM system is preferred for the machining process.

The CAD/CAM system is not particularly limited, and known devices may be used. Examples of such devices include known CAD/CAM systems such as the KATANA® CAD/CAM system manufactured by Kuraray Noritake Dental Inc.

The present invention encompasses embodiments combining all or part of the foregoing features in various ways within the technical idea of the present invention, provided that the present invention can exhibit its effects.

EXAMPLES

The present invention is described below in greater detail through Examples. It is to be noted, however, that the present invention is in no way limited by the following Examples, and various modifications may be made by a person with ordinary skill in the art within the technical idea of the present invention.

[Example 1]

A commercially available monoclinic zirconia powder ($Y_2O_3$: 0 mol%) and a commercially available yttria powder were charged into water at a concentration of 20 to 30 mass% (a yttria content of 4.5 mol% in terms of the mole percentage of yttria relative to the total number of moles of zirconia and yttria). The mixture, along with a pulverization medium made of zirconia (diameter: 0.1 mm), was placed in the vessel of a bead mill, and pulverized for 1 hour with the bead mill to obtain a slurry.

The slurry was then dried with a dryer at 85°C. The dried powder, with moisture removed, was added into ethanol in a sample vial to a concentration of approximately 0.1 mass%, and subjected to ultrasonic dispersion for 2 to 30 minutes, producing an ethanol-diluted slurry containing the zirconia composition.

<TEM Observation of Zirconia Composition>

One or two drops of the ethanol-diluted slurry was applied to a TEM microgrid (Microgrid Mo100P, 250 mesh equivalent, manufactured by STEM under the trade name NP-M10-25) and allowed to dry. After confirming that the powder had been mounted on the grid under a light microscope, measurement was conducted using a TEM (an atomic-resolution analytical electron microscope manufactured by JEOL Ltd. under the trade name JEM-ARM200F ACCELARM) at an accelerating voltage of 200 kV, with 200,000 or 1500,000× magnification. The magnification can be adjusted as appropriate according to the particle size of the object under observation.

<Method for Identifying Zirconia Particles>

Zirconia particles (with a zirconia concentration of 80 mass% or more) were identified using the following procedure.
Under bright-field TEM observation at high magnification (200,000×), particles containing an observation point (a $5 \times 5$ nm observation area) with a zirconia concentration of 80 mass% or more as determined by scanning transmission electron microscopy with energy dispersive X-ray spectroscopy (STEM-EDS) and visually appearing nearly uniform in brightness were regarded as single zirconia particles. Alternatively, in dark-field, an accelerating voltage that produces a clear diffraction image of monoclinic zirconia was applied, and particles exhibiting increased brightness were regarded as single zirconia particles.

<Method for Measuring Average Primary Particle Diameter of Zirconia Particles>

The equivalent circular diameter (area-equivalent circular diameter) of each particle, identified using the previously described method for identifying zirconia particles, was taken as its primary particle diameter, and the mean value of the equivalent circular diameters of ten arbitrarily selected particles in a single field at 200,000× magnification was calculated as the average primary particle diameter of the zirconia particles.

<Method for Identifying Small Particles>

The small particles were identified using the following procedure.
Using the TEM employed in the foregoing section <TEM Observation of Zirconia Composition>, particles smaller than the average primary particle diameter of zirconia particles were examined under bright-field TEM at ultra-high magnification (1,500,000×). Focusing on a single particle, regions with visually observable interruptions (or shifts) in lattice fringes were regarded as grain boundaries, and each such region was considered a single small particle.

<Method for Measuring Primary Particle Diameter and Average Primary Particle Diameter of Small Particles>

The equivalent circular diameter (area-equivalent circular diameter) of each particle, identified using the previously described method for identifying small particles, was taken as its primary particle diameter, and the mean value of the equivalent circular diameters of ten arbitrarily selected particles in a single field at 200,000× magnification was calculated as the average primary particle diameter of the small particles.

<Method for Measuring Yttria Concentration of Small Particles>

The yttria concentration of each individual small particle, identified using the previously described method for identifying small particles, was measured by STEM-EDS (over a $5 \times 5$ nm observation area).
In Example 1, the yttria concentration fell within the 10 to 50 mass% range in all of ten arbitrarily selected small particles from a single field observed at 200,000× magnification.

<Method for Measuring the Area Ratio of Zirconia Particles and Small Particles>

From particles identified using the previously described methods for identifying zirconia particles and small particles, the area ratio (%) of zirconia particles and small particles was calculated based on the areas of ten arbitrarily selected zirconia particles and the areas of ten small particles arbitrarily selected within a single field at 200,000× magnification.

An organic binder was added into the ethanol-diluted slurry obtained in the manner described above. After mixing and stirring, the slurry was dried and granulated with a spray dryer to obtain a powder. The powder was poured into a columnar mold, uniaxially pressed at 33 MPa pressure, and subjected to CIP at 190 MPa to obtain a plate- or disc-shaped molded body.

The molded body was placed in an electric furnace, heated from room temperature at 10°C/min, and retained at 500°C for 2 hours to debind the organic components. The resulting material was then heated at 10°C/min, held at 875°C for 2 hours, and allowed to cool at -10°C/min to obtain a zirconia pre-sintered body.

The zirconia pre-sintered body was fired under ordinary pressure by employing sintering conditions with a rate of temperature increase of 10°C/min, a highest sintering temperature of 1,375°C, and a hold time of 2 hours, yielding a zirconia sintered body containing 4.5 mol% yttria. The zirconia sintered body produced was white in color.

[Comparative Example 1]

The zirconia pre-sintered body and zirconia sintered body according to Comparative Example 1 were produced following the same method employed in Example 1, except that the pulverization process was carried out for 20 hours using a ball mill (diameter: 2 mm), instead of the bead mill.

<Three-Point Flexural Strength>

The three-point flexural strength of the zirconia sintered body was measured in compliance with ISO 6872:2015.

From the plate-shaped zirconia sintered body of each Example and Comparative Example, samples each measuring 4 mm × 1.2 mm × 15 mm were prepared. These samples were then measured using a universal testing machine at a span length of 12 mm and a crosshead speed of 0.5 mm/min.

<Opalescence (OP value) of Zirconia Sintered Body>

The OP value of the zirconia sintered body was determined by measuring $a^*_{transmission}$, $a^*_{reflection}$, $b^*_{transmission}$, and $b^*_{reflection}$ using a spectrophotometer (CM-3610A manufactured by Konica Minolta Japan), followed by calculation using the following formula.

[Math. 1]

$$\text{OP value} = \sqrt{\left(a^*_{transmission} - a^*_{reflection}\right)^2 + \left(b^*_{transmission} - b^*_{reflection}\right)^2}$$

In the measurement, the reflected light was measured using an F11 light source. For the measurement, a disc-shaped zirconia sintered body, measuring 15 mm in diameter and 1.0 mm in thickness, was used as the sample after polishing the both surfaces to mirror finish. The measurement was performed for n = 3, and the mean value was calculated.

An OP value of 4.5 or more and 9.0 or less was considered acceptable.

[Table 1]

| | | Zirconia composition | | | | | Zirconia sintered body | |
|---|---|---|---|---|---|---|---|---|
| | | Zirconia concentration of zirconia particles (mass%) | Yttria concentration of small particles (mass%) | Average primary particle diameter of zirconia particles (nm) | Average primary particle diameter of small particles (nm) | Area percentage of small particles (%) | Three-point flexural strength [MPa] | Opalescence |
| | Example 1 | 88.5, 90.3, 91.1, 93.4, 96.6, 97.4, 98.3, 98.8, 99.1, 99.6 | 11.5, 11.5, 15.8, 15.8, 18.8, 19.6, 20.3, 23.2, 35.3, 44.0 | 72 | 31 | 16 | 1104 | 7.5 |

(continued)

| | Zirconia composition | | | | | Zirconia sintered body | |
|---|---|---|---|---|---|---|---|
| | Zirconia concentration of zirconia particles (mass%) | Yttria concentration of small particles (mass%) | Average primary particle diameter of zirconia particles (nm) | Average primary particle diameter of small particles (nm) | Area percentage of small particles (%) | Three-point flexural strength [MPa] | Opalescence |
| Comparative Example 1 | 89.3, 92.3, 95.3, 95.4, 95.8, 97.3, 97.6, 98.0, 98.4, 99.2 | 86.6, 85.3, 87.2, 88.0, 89.5, 90.2, 90.4, 91.9, 93.4, 96.7 | 89 | 45 | 20 | 957 | 3.1 |

Referring to the table, the yttria content of the sintered body was 4.5 mol% in Example 1 and Comparative Example 1.

The zirconia concentration (mass%) in small particles can be calculated from the yttria concentration of small particles presented in Table 1 by subtracting the yttria concentration (mass%) from 100 (mass%).

These results confirmed that the zirconia pre-sintered body of the present invention can yield a zirconia sintered body with excellent mechanical strength while effectively controlling the opalescence within an appropriate range.

Comparative Example1, representing a zirconia composition that does not contain small particles with a stabilizer concentration of 10 to 50 mass%, failed to control the opalescence within an appropriate range and achieve the desired properties.

INDUSTRIAL APPLICABILITY

A zirconia composition of the present invention is useful for the production of dental products (particularly, those used in dental treatments at dental clinics).

**Claims**

1. A zirconia composition comprising:

   zirconia particles with a zirconia concentration of 80 mass% or more; and
   small particles with a concentration of 10 to 50 mass% of a stabilizer capable of preventing a phase transformation of zirconia,
   wherein the small particles have a primary particle diameter smaller than the average primary particle diameter of the zirconia particles.

2. The zirconia composition according to claim 1, wherein the small particles with a stabilizer concentration of 10 to 50 mass% represent six or more of ten small particles arbitrarily selected under observation by TEM.

3. The zirconia composition according to claim 1 or 2, wherein the average primary particle diameter of the zirconia particles is 0.06 to 0.17 $\mu$m.

4. The zirconia composition according to claim 1 or 2, wherein the small particles have an average primary particle diameter of less than 0.06 $\mu$m.

5. The zirconia composition according to claim 1 or 2, wherein the stabilizer capable of preventing a phase transformation of zirconia is yttria.

6. The zirconia composition according to claim 1 or 2, wherein the area ratio of the zirconia particles and the small particles is 55/45 to 99/1 under observation by TEM.

7. A method for producing a zirconia composition of claim 1 or 2, comprising the steps of:

   producing a raw material composition comprising zirconia particles, and particles of a stabilizer capable of preventing a phase transformation of zirconia; and
   pulverizing the zirconia composition,
   wherein the pulverization step employs a pulverization medium of less than 1 mm in diameter.

8. The method for producing a zirconia composition according to claim 7, wherein the pulverization step involves a processing time of 20 minutes to 2 hours.

9. The method for producing a zirconia composition according to claim 7, wherein the average primary particle diameter of the zirconia particles is 0.06 to 0.17 $\mu$m.

10. The method for producing a zirconia composition according to claim 7, wherein the small particles have an average primary particle diameter of less than 0.06 $\mu$m.

11. The method for producing a zirconia composition according to claim 7, wherein the stabilizer capable of preventing a phase transformation of zirconia is yttria.

12. The method for producing a zirconia composition according to claim 7, which further comprises the step of producing a slurry comprising the raw material composition and granulating the slurry by spray drying.

## INTERNATIONAL SEARCH REPORT

| International application No. |
| --- |
| **PCT/JP2024/033776** |

| A. | CLASSIFICATION OF SUBJECT MATTER |
| --- | --- |

*C04B 35/486*(2006.01)i; *A61C 13/083*(2006.01)i; *A61K 6/818*(2020.01)i; *A61K 6/824*(2020.01)i; *C01G 25/02*(2006.01)i
FI:    C04B35/486; A61K6/818; A61K6/824; C01G25/02; A61C13/083

According to International Patent Classification (IPC) or to both national classification and IPC

| B. | FIELDS SEARCHED |
| --- | --- |

Minimum documentation searched (classification system followed by classification symbols)

C04B35/00-C04B35/84; A61C13/083; A61K6/818; A61K6/824; C01G25/00-C01G25/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2023/120674 A1 (KURARAY NORITAKE DENTAL INC.) 29 June 2023 (2023-06-29) claims, paragraphs [0147]-[0185] | 1-6 |
| A | | 7-12 |
| X | JP 2015-196624 A (TOSOH CORP.) 09 November 2015 (2015-11-09) claims, paragraphs [0057]-[0075] | 1-6 |
| A | | 7-12 |
| A | JP 59-107966 A (TOSHIBA CERAMICS CO.) 22 June 1984 (1984-06-22) | 1-12 |
| A | JP 7-165465 A (TIOXIDE SPECIALTIES LTD.) 27 June 1995 (1995-06-27) | 1-12 |
| A | JP 6-510738 A (ROBERT BOSCH GMBH) 01 December 1994 (1994-12-01) | 1-12 |
| A | JP 2015-516351 A (FRAUNHOFER-GESELLSCHAFT ZUR FOERDERUNG DER ANGEWANDTEN FORSCHUNG E.V.) 11 June 2015 (2015-06-11) | 1-12 |

☐ Further documents are listed in the continuation of Box C.        ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **25 October 2024** | **19 November 2024** |

| Name and mailing address of the ISA/JP | Authorized officer |
| --- | --- |
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

**INTERNATIONAL SEARCH REPORT**
Information on patent family members

International application No.

**PCT/JP2024/033776**

| Patent document cited in search report | | | Publication date (day/month/year) | Patent family member(s) | | | Publication date (day/month/year) |
|---|---|---|---|---|---|---|---|
| WO | 2023/120674 | A1 | 29 June 2023 | KR 10-2024-0099440 | | A | |
| JP | 2015-196624 | A | 09 November 2015 | (Family: none) | | | |
| JP | 59-107966 | A | 22 June 1984 | (Family: none) | | | |
| JP | 7-165465 | A | 27 June 1995 | EP | 637576 | A2 | |
| JP | 6-510738 | A | 01 December 1994 | US | 5681784 | A | |
| JP | 2015-516351 | A | 11 June 2015 | US | 2015/0118648 | A1 | |
| | | | | CN | 104159870 | A | |

Form PCT/ISA/210 (patent family annex) (July 2022)

**REFERENCES CITED IN THE DESCRIPTION**

**Patent documents cited in the description**

- WO 2022138881 A